# EUROPEAN PATENT SPECIFICATION

(11) **EP 0 724 421 B1**
(45) Date of publication and mention of the grant of the patent: **15.04.1998**
(21) Application number: 94929715.4
(22) Date of filing: 06.10.1994
(51) Int. Cl.: A61F 13/74

(54) **A SANITARY PANTIE**
HYGIENISCHE UNTERHOSE
CULOTTE HYGIENIQUE

(30) Priority: 07.10.1993 SE 9303283
(43) Date of publication of application: 07.08.1996
(73) Proprietor: SCA Mölnlycke AB, 405 03 Göteborg (SE)
(72) Inventor: JOHANSSON, Kerstin, S-523 38 Ulricehamn (SE)
(74) Representative: Hyltner, Jan-Olof
(86) International application number: SE9400932
(87) International publication number: WO9509594

(56) References cited:
- WO-A-90/09159
- DE-A- 3 740 002

## Description

The present invention relates to a sanitary pantie or to a light-incontinence guard comprising a back-part, a front-part, an intermediate crotch-part and elastic devices which extend between the front-part and the back-part of the pantie and which function to press an absorbent body permanently mounted or removably attached to the pantie into abutment with the wearer's body.

SE-B-434,006 describes a sanitary pantie of this kind which includes a plurality of mutually parallel elastic threads which extend in a straight line between the front and back parts of the pantie. The object of the pantie according to this publication is to eliminate the drawbacks of sanitary panties that are produced entirely of elastic material, by causing the uniformly distributed elastic threads to press a sanitary napkin or like article carried in the pantie into essentially uniform abutment with the wearer's body. However, the known pantie has not been found to provide a completely satisfactory solution to the problem of providing a sanitary pantie which is comfortable to wear and which prevents leakage from an absorbent body or pad carried by the pantie.

DE-A-3 740 002 discloses a diaper according to the preamble of claim 1. However, nothing is mentioned in this document about how to construct elastic devices for sanitary napkins or sanitary panties.

The object of the present invention is to solve this problem more effectively than known sanitary panties and to provide a sanitary pantie which is comfortable to wear and which is highly effective in preventing leakage from an absorbent body carried by the pantie.

This object is achieved in accordance with the invention by means of a sanitary pantie of the kind defined in the introduction which is characterized in that two elastic devices extend from a region in the crotch-part of the pantie to the front-edge of the front-part of the pantie while diverging in relation to one another and extending generally symmetrically in relation to a centre line through the back-part, the crotch-part and the front-part of the pantie. In addition to pressing corresponding parts of the absorbent body into abutment with the wearer's body within the region in which the elastic devices extend, the elastic devices also form the absorbent body into a shape which conforms with the anatomy of the wearer, wherein those parts of the absorbent body which lie between neighbouring elastic devices will also conform well to the wearer's anatomy.

According to one preferred embodiment of the inventive pantie, at least one elastic device extends from that region which is located in the diaphragm or midriff region of the wearer in use extends to the rear-edge of the back-part of the pantie essentially along a central line which extends through the front-part, the crotch-part and the back-part of the pantie. Preferably, further elastic devices extend divergently relative to one another and symmetrically in relation to a central line passing through the front-part, the crotch-part and the back-part of the pantie, from the centre-part of said crotch-part to the rear-edge of the back-part and to the front-edge of said front-part. The outermost elastic devices of said further elastic devices in relation to said central line extend along the side-edges of an absorbent body that coacts with the pantie, at least within the centre-part of the crotch-part. The elastic devices may conveniently comprise elastic threads, ribbons or bands and the pantie may be comprised of two layers or sheets with the elastic devices mounted therebetween. The aforesaid two sheets are preferably joined together and to the elastic devices by gluing or ultrasonic welding.

Exemplifying embodiments of an inventive sanitary pantie will now be described with reference to the accompanying drawings, in which
Figure 1 is a view of a first embodiment of an inventive sanitary pantie taken from above, before fastening the waist-parts of the pantie together;
Figures 2 and 3 are front and rear views respectively showing the sanitary pantie of Figure 1 when assembled and when worn;
Figure 4 is a schematic illustration showing deformation of an absorbent body inserted in a pantie worn by a user; and
Figure 5 is a view similar to Figure 1 illustrating a second embodiment of an inventive sanitary pantie.

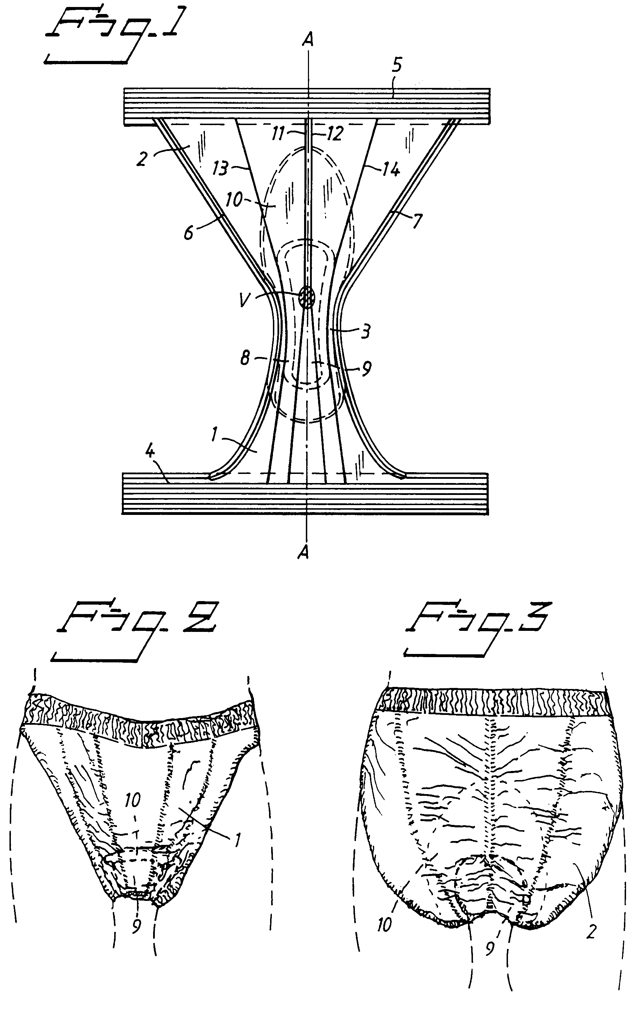

Figure 1 illustrates a sanitary pantie according to one embodiment of the invention and shows the pantie in a stage of manufacture prior to joining together the front and the rear waist-parts. The illustrated pantie includes a front-part 1, a back-part 2 and an intermediate crotch-part 3. The pantie is also provided conventionally with waist elastic, which in the illustrated case has the form of elastic ribbon 4, 5 mounted along the front-part and the back-part of the waist edge, and leg elastic in the form of elastic threads 6, 7 extending along the side-contours of the pantie between the elastic ribbons 4, 5. In the illustrated case, two elastic threads are mounted along each side-edge of the pantie, although it will be understood that the leg elastic may, instead, consist of more or fewer threads than shown or of elastic ribbons or the like. Figure 1 also shows an absorbent body or pad 8 in broken lines, which may be either attached firmly to the pantie or removably joined thereto. The illustrated absorbent body 8 is comprised of a primary absorbent body 9 and a secondary absorbent body 10 and is particularly suited for night use.

The pantie is also provided with two elastic threads 11, 12 which extend between the respective elastic ribbons 4 and 5 of the front and back parts 1, 2, symmetrically in relation to a centre line A-A extending in the longitudinal direction of the pantie. The threads 11, 12 extend close together and parallel with the longitudinal pantie line, from the waist-edge of the back-part 2 or the rear-edge of said back-part to a crotch region V, which when the pantie is worn is located in the midriff region of the wearer. The threads 11, 12 extend divergently from this region V to the waist-edge or the front-edge of the front-part of the pantie.

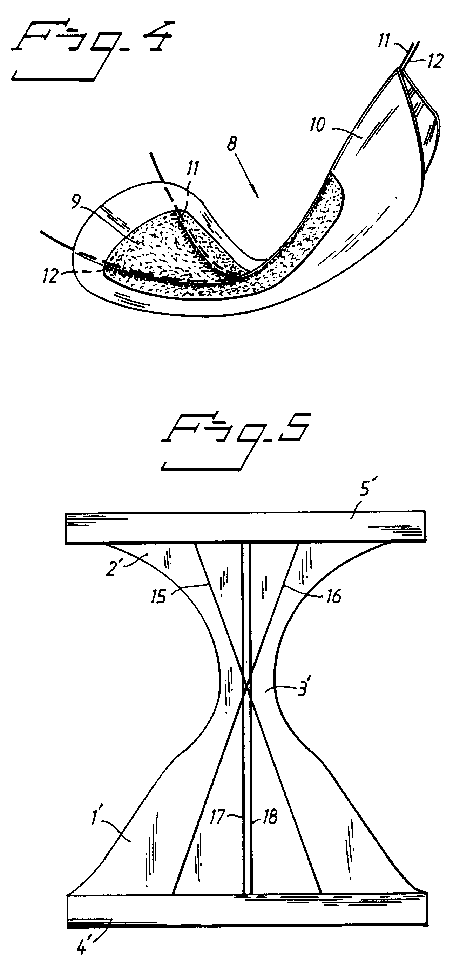

When wearing a pantie constructed in accordance with the Figure 1 embodiment, those parts of the threads 11, 12 which diverge forwardly from the region V strive to bring the absorbent body 8 to a basin-like shape within this region, this basin-like shape corresponding well to the wearer's anatomy within this region. Those mutually parallel parts of the threads 11, 12 which extend rearwardly from the region V strive to press corresponding central parts of the absorbent body 8 in between the wearer's buttocks when the pantie is worn, thereby causing the absorbent body to lie safely against the wearer's body, at least within the crotch-part and the beginning of the back-part of the pantie, so as to provide effective safety against rearward leakage. The aforementioned shaping of the absorbent body is illustrated schematically in Figure 4.

The pantie illustrated in Figure 1 also includes elastic threads 13, 14 which lie laterally outside the threads 11, 12 and which are mutually convergent between the front-part and the back-part of the pantie and are mutually divergent in the back part of the pantie. The elastic threads 13, 14 follow the side contours of the primary absorbent body 9, along a major part of their length extension. The threads 13, 14 thus ensure that the side-edges of the primary absorbent body will be pressed into sealing abutment with the wearer's body and also contribute towards ensuring that the secondary absorbent body will follow the wearer's body effectively. It should be mentioned in this connection that the secondary absorbent body is thin and very pliable and is intended to form an additional safety zone for absorbing any leakage that may occur during long-term use of the absorbent body, for instance over a full night, and that its absorption capacity may be relatively small.

When the absorbent body that coacts with the sanitary pantie is to form an integral part of the pantie, the absorbent body is preferably anchored to the pantie in the stage of manufacture illustrated in Figure 1, in which the aforedescribed elastic elements are in a stretched state. When manufacture of the pantie is completed, the elastic elements strive to contract to a relaxed or tensionless state. This results in the formation of folds in those regions of the pantie which lie outside the primary absorbent body 9, and the threads will contract to a generally relaxed state within these regions. Within the region of the primary absorbent body 9, the absorbent body counteracts gathering of the pantie into folds, to a greater or lesser extent dependent on the stiffness thereof. Present-day thin absorbent bodies are extremely pliable and will therefore be folded by the elastic threads, although the threads are prevented from relaxing totally. Thus, when the pantie is put on the wearer's body, the folds in the absorbent body 9 will be smoothed out and the body will be brought into abutment with the wearer's body by the elastic force in respective threads 11-14. The absorbent body 9 will obtain a concave basin-like shape from the region V and forwards, due to the wearer's anatomy, causing the contracting force exerted by the threads 11-14 within this region to strive to retain this basin-like shape while, at the same time, those parts of the absorbent body 9 which lie along the threads will be pressed into sealing abutment with the wearer's body. The mutually parallel parts of the threads 11, 12 ensure that the part of the absorbent body 9 that lies rearwards of the region V will be deformed so as to conform to the shape of the wearer's anatomy in this region and so that the part of the absorbent body that lies between the wearer's buttocks will come into sealing abutment with the wearer's body. In addition to ensuring that the side-edges of the absorbent body 9 will sealingly abut the wearer's body, the elastic threads 13, 14 also function to prevent the formation of folds or buckles in the pantie or in the secondary absorbent body.

When the pantie illustrated in Figure 1 is intended to be used together with a removable absorbent body, the absorbent body should be inserted with the elastic threads outwardly stretched at least within the region of attachment of the primary absorbent body. A pantie of this kind will function essentially in the manner described above with reference to an integrated absorbent body. This is also a natural method of attaching the absorbent body, since folds would otherwise form in the crotch-part of the pantie. It should be mentioned in this connection that a removable absorbent body which is intended to coact with the inventive pantie will preferably have a stiffness such as not to be folded by the elastic threads after attachment to the pantie, but merely curved.

Figures 2 and 3 illustrate schematically the form taken by the sanitary pantie of Figure 1 when worn, the relevant parts of the wearer's body being shown in broken lines.

In the case of the Figure 1 embodiment, the elastic devices 11-14 are elastic threads. It will be understood, however, that the necessary elasticity of the pantie can be achieved in ways other than with the aid of elastic threads. For instance, the elastic devices may have the form of elastic bands, ribbons or elastic film, or nonwoven material cut to the desired shape. It is also conceivable to produce the pantie from an elastic material and to remove the elastic properties of the material in some suitable way within desired regions or areas. Different types of elastic material may be combined in the illustrated pantie. For instance, soft bands or ribbons of elastic foam may be used as leg elastic, while complete pieces of elastic nonwoven material may be used for the waist elastic. It is also conceivable for the elastic devices 11-14 to comprise parts of mutually different materials. For instance, the parallel parts of the threads 11, 12 can be replaced with a centrally extending band while the diverging parts are comprised of elastic threads.

The pantie illustrated in Figure 1 is preferably comprised of two sheets which are mutually joined in some appropriate manner, for instance glued, and the elastic devices are mounted between these sheets and fastened thereto, for instance glued or welded with ultrasonic welds or heat welds. The elastic may also be sewn in the pantie.

As before mentioned, the absorbent body 8 illustrated in Figure 1 is comprised of a primary and a secondary absorbent body 9 and 10 respectively. The primary absorbent body 9 is constructed in the same manner as a conventional absorbent body or pad of a sanitary napkin and will have an absorbency sufficient to handle fluid discharged by the wearer over the intended use time-period, for instance a full night. For instance, the absorbent body may be comprised of one or more layers of compressed cellulose fluff which may or may not contain superabsorbent material. The absorbent body 9 is advantageously comprised of one or more layers of roll material, i.e. absorbent material which has earlier been compressed and treated so that the materials can be stored in the form of rolls. Such absorbent bodies may be very thin and pliable while still having sufficiently high absorbency for use as a night napkin. The secondary absorbent body 10 has much larger extensions than the primary absorbent body and extends rearwardly over a larger part of the back-part of the pantie. The primary purpose of the secondary absorbent body is to enhance leakage safety and shall therefore have a given absorbency. To this end, the secondary absorbent body may be comprised of tissue, nonwoven or other roll material. The secondary absorbent body 10 will also preferably contain means for preventing liquid from spreading over the surface of said body. Such fluid-spreading barriers may be obtained, for instance, by folding, pleating or crêping the material sheet or layer of said body, or by providing barrier-type welds. It is also conceivable to use a nonwoven material which includes fibres of so-called superabsorbent material which, when absorbing fluid, bind the absorbed fluid chemically. However, the secondary absorbent body will, in general, be equally as pliable and supple as the pantie material, so that the pantie can be worn comfortably. The secondary absorbent body must therefore be made very thin and will subsequently have highly limited absorbency.

The combined absorbent body 8, comprising the primary and the secondary absorbent bodies, is enclosed conventionally between a fluid-permeable casing sheet and a fluid-impermeable but advantageously air-permeable casing sheet which lies against the pantie in use.

As will be seen from Figure 1, the side-contours of the secondary absorbent body follow the side-contours of the pantie within the region of the narrowest part of the crotch-part. This is highly advantageous when the combined absorbent body 8 is a removable type body, since its configuration clearly indicates where the absorbent body 8 shall be placed in the pantie. This eliminates to a large extent the risk of a user placing the absorbent body wrongly in the pantie.

As will be understood, the described pantie can be used together with absorbent bodies other than the absorbent body described with reference to the exemplifying embodiment, such as an absorbent body which lacks a secondary absorbent body of the aforedescribed kind. The absorbent body may also have a shape different to that shown in Figure 1, for instance a rectangular shape.

Figure 5 shows a second embodiment of an inventive sanitary pantie. Those pantie components of the Figure 5 embodiment which find correspondence in the pantie illustrated in Figure 1 have been identified by the same reference signs to which a prime has been added.

In the case of the pantie illustrated in Figure 5, two elastic threads 15, 16 extend obliquely and symmetrically relative to a central long line through the front-part, the crotch-part and the back-part of the pantie, from the front-edge of the front-part 1' to the rear-edge of the back-part 2'. The threads 15, 16 cross one another at a point located in the midriff region of a user wearing the pantie. Similar to the pantie illustrated in Figure 1, the pantie includes two threads which diverge away from one another in a forward direction from the midriff point. These parts of the threads 15, 16 act in a manner analogous with the aforedescribed corresponding thread-parts of the Figure 1 embodiment when wearing the pantie with an inserted absorbent body.

The threads 15, 16 can be supplemented with threads 17, 18 which extend parallel with one another close to the aforesaid central long line, so as to cause a coacting absorbent body rearwardly of the midriff point to be pressed in against the wearer's body between the buttocks thereof.

It will be understood that the aforedescribed embodiments can be modified in several ways within the scope of the invention. For instance, the pantie may be constructed from one single material sheet. The pantie may also include transverse elastics within the region of the primary absorbent body, particularly when the absorbent body is integral with the pantie, so as to deform an inserted absorbent body in the manner desired. In the case of removable absorbent bodies, the absorbent bodies may also be fastened to the pantie within the region of such transverse elastic. Further elastic threads may also be mounted between the laterally outermost threads 13 and 14, these threads also extending in a convergent-divergent pattern in relation to one another. An absorbent layer corresponding to the secondary absorbent body in the pantie of Figure 1 may also be formed integrally with the pantie. In this case, an absorbent body coacting with a pantie of this nature need only be enclosed in a fluid-permeable casing layer. The invention is therefore limited solely by the content of the following Claims.

## Claims

1. A sanitary pantie or a light-incontinence guard comprising a back-part (2; 2'), a front-part (1; 1'), an intermediate crotch-part (3; 3') and elastic devices (11-14; 15-18) which extend between the front-part and the back-part of the pantie and which function to press an absorbent body (8) permanently mounted or removably attached to the pantie into abutment with the wearer's body, **characterized** in that two elastic devices (11, 12; 15, 16) extend from a region (V) in the crotch-part of the pantie to the front-edge of the front pantie-part (1; 1') while diverging relative to one another and extending generally symmetrically in relation to a central line (A-A) which extends through the back-part (2; 2'), the crotch-part (3; 3') and the front-part (1; 1') of the pantie.

2. A pantie according to Claim 1, **characterized** in that at least one elastic device (11, 12; 17, 18) extends from the region (V) in the crotch-part to the rear edge of the back pantie-part (2; 2') generally along a central line (A-A) which extends through the front-part (1; 1'), the crotch-part (3; 3') and the back-part (2; 2') of the pantie.

3. A pantie according to Claim 1 or 2, **characterized** in that at least two elastic devices (15, 16) extend from the crotch-part region to the rear-edge of the back pantie-part (2') while diverging relative to one another and extending symmetrically in relation to a centre line which passes through the back-part (2'), the crotch-part (3') and the front-part (1') of the pantie.

4. A pantie according to any one of the preceding Claims, **characterized** in that further elastic devices (13, 14) extend divergently in relation to one another and symmetrically in relation to a central line (A-A) which passes through the front-part (1), the crotch-part (3) and the back-part (2) of the pantie, from the centre part of the crotch-part (3) to the rear-edge of the back-part (2) and to the front-edge of the front pantie-part (1).

5. A pantie according to Claim 4, **characterized** in that the further elastic devices (13, 14) that lie outermost in relation to said central line (A-A) extend along the side-edges of an absorbent body (8) coacting with the pantie, at least within the centre part of the crotch-part (3).

6. A pantie according to any one of the preceding Claims, **characterized** in that the elastic devices (11-14; 15-18) are comprised of elastic threads, bands or ribbons.

7. A pantie according to any one of the preceding Claims, **characterized** in that the pantie is comprised of two sheets or layers, and in that the elastic devices (11-14; 15-18) are mounted between said two sheets or layers.

8. A pantie according to Claim 7, **characterized** in that the two pantie sheets or layers are joined to one another and to the elastic devices (11-14; 15-18) by gluing or ultrasonic welding.

## Patentansprüche

1. Hygieneunterhose oder Schutz gegen leichte Inkontinenz umfassend einen Rückteil (2; 2'), ein Vorderteil (1; 1'), ein zwischenliegendes Schritteil (3; 3') und elastische Einrichtungen (11-14; 15-18), die sich zwischen dem Vorderteil und dem Rückteil der Unterhose erstrecken und die dazu dienen, einen dauerhaft oder lösbar an der Unterhose befestigten Absorptionskörper (8) an den Körper des Trägers zu drücken, **dadurch gekennzeichnet, daß** sich zwei elastische Einrichtungen (11, 12; 15, 16) von einem Bereich (V) im Schritteil der Unterhose zu dem vorderen Rand des Vorderteils der Unterhose (1; 1') erstrecken, wobei sie relativ zueinander auseinanderlaufen und sich im wesentlichen symmetrisch zu einer sich durch den Rückteil (2; 2'), den Schritteil (3; 3') und den Vorderteil (1; 1') der Unterhose erstreckenden Mittellinie (A-A) erstrecken.

2. Unterhose nach Anspruch 1, **dadurch gekennzeichnet, daß** sich zumindest eine elastische Einrichtung (11, 12; 16, 17) im wesentlichen entlang einer sich durch den Vorderteil (1; 1'), den Schritteil (3; 3') und den Rückteil (2; 2') der Unterhose erstreckenden Mittellinie (A-A) von dem Bereich (V) im Schritteil zu der Hinterkante des Rückteils (2; 2') der Unterhose erstreckt.

3. Unterhose nach Anspruch 1 oder 2, **dadurch gekennzeichnet, daß** sich zumindest zwei elastische Einrichtungen (15, 16) von dem Schritteilbereich zu der Hinterkante des Rückteils (2') der Unterhose erstrecken, wobei sie relativ zueinander auseinanderlaufen und sich im wesentlichen symmetrisch zu einer sich durch den Rückteil (2'), den Schritteil (3') und den Vorderteil (1') der Unterhose erstreckenden Mittellinie (A-A) erstrecken.

4. Unterhose nach einem der vorhergehenden Ansprüche, **dadurch gekennzeichnet, daß** sich weitere elastische Einrichtungen (13, 14) voneinander auseinandergehend und symmetrisch zu einer sich durch den Vorderteil (1), den Schritteil (3) und den Rückteil (2) der Unterhose erstreckenden Mittellinie (A-A) von dem Mittelabschnitt des Schritteils (3) zu der Hinterkante des Rückteils (2) und zu der Vorderkante des Vorderteils (1) der Unterhose erstrecken.

5. Unterhose nach Anspruch 4, **dadurch** g**ekennzeichnet, daß** die weiteren elastischen Einrichtungen (13, 14), die bezüglich der Mittellinie (A-A) am weitesten außen liegen, sich zumindest im Mittelabschnitt des Schritteils (3) entlang der Seitenränder eines mit der Unterhose zusammenwirkenden Absorptionskörpers (8) erstrecken.

6. Unterhose nach einem der vorhergehenden Ansprüche, **dadurch gekennzeichnet, daß** die elastischen Einrichtungen (11-14; 15-18) aus Gummifäden, -bändern oder -streifen bestehen.

7. Unterhose nach einem der vorhergehenden Ansprüche, **dadurch gekennzeichnet, daß** die Unterhose aus zwei Lagen oder Schichten besteht und daß die elastischen Einrichtungen (11-14; 15-18) zwischen diesen zwei Lagen oder Schichten angebracht sind.

8. Unterhose nach Anspruch 7, **dadurch gekennzeichnet, daß** die zwei Schichten oder Lagen der Unterhose miteinander und mit den elastischen Einrichtungen (11-14; 15-18) durch Verkleben oder Ultraschallschweißen verbunden sind.

## Revendications

1. Culotte hygiénique ou protection contre l'incontinence légère, comprenant une partie arrière (2 ; 2'), une partie avant (1 ; 1'), une partie intermédiaire d'entre-jambes (3 ; 3') et des dispositifs élastiques (11-14 ; 15-18) qui s'étendent entre la partie avant et la partie arrière de la culotte et qui servent à pousser un corps absorbant (8), monté de manière permanente ou fixé de manière amovible a la culotte, contre le corps de l'utilisateur, caractérisée en ce que deux dispositifs élastiques (11, 12; 15, 16) s'étendent d'une région (V) dans la partie d'entre-jambes de la culotte au bord avant de la partie avant (1 ; 1') de la culotte, en divergeant l'un par rapport à l'autre et en s'étendant de manière globalement symétrique par rapport à une ligne centrale (A-A) qui s'étend dans la partie arrière (2 ; 2'), la partie d'entre-jambes (3 ; 3') et la partie avant (1 ; 1') de la culotte.

2. Culotte selon la revendication 1, caractérisée en ce qu'au moins un dispositif élastique (11, 12 ; 17, 18) s'étend de la région (V) dans la partie d'entre-jambes au bord arrière de la partie arrière (2 ; 2') de la culotte, globalement le long d'une ligne centrale (A-A) qui s'étend dans la partie avant (1 ; 1'), la partie d'entre-jambes (3 ; 3') et la partie arrière (2 ; 2') de la culotte.

3. Culotte selon la revendication 1 ou 2, caractérisée en ce qu'au moins deux dispositifs élastiques (15, 16) s'étendent de la partie d'entre-jambes au bord arrière de la partie arrière (2') de la culotte, en divergeant l'un par rapport à l'autre et en s'étendant de manière globalement symétrique par rapport à une ligne centrale (A-A) qui s'étend dans la partie arrière (2'), la partie d'entre-jambes (3') et la partie avant (1') de la culotte.

4. Culotte suivant l'une quelconque des revendications précédentes, caractérisée en ce que d'autres dispositifs élastiques (13, 14) s'étendent en divergeant l'un par rapport à l'autre et de manière symétrique par rapport à une ligne centrale (A-A) qui s'étend dans la partie avant (1), la partie d'entre-jambes (3) et la partie arrière (2) de la culotte, de la région centrale de la partie d'entre-jambes (3) au bord arrière de la partie arrière (2) de la culotte et au bord avant de la partie avant (1) de la culotte.

5. Culotte selon la revendication 4, caractérisée en ce que les autres dispositifs élastiques (13, 14) qui sont placés les plus a l'extérieur par rapport à ladite ligne centrale (A-A) s'étendent le long des bords latéraux d'un corps absorbant (8) coopérant avec la culotte, au moins dans la région centrale de la partie d'entre-jambes (3).

6. Culotte suivant l'une quelconque des revendications précédentes, caractérisée en ce que les dispositifs élastiques (11-14 ; 15-18) sont faits de rubans, bandes ou fils élastiques.

7. Culotte suivant l'une quelconque des revendications précédentes, caractérisée en ce que la culotte est faite de deux feuilles ou couches, et en ce que les dispositifs élastiques (11-14 ; 15-18) sont montés entre lesdites deux feuilles ou couches.

8. Culotte selon la revendication 7, caractérisée en ce que les deux feuilles ou couches de la culotte sont réunies l'une a' l'autre et aux dispositifs élastiques (11-14 ; 15-18) par collage ou par soudage aux ultrasons.
